# FASCICULE DE BREVET EUROPEEN

(11) **EP 0 437 141 B1**
(45) Date de publication et mention de la délivrance du brevet: **03.04.1996**
(21) Numéro de dépôt: 90403744.7
(22) Date de dépôt: 21.12.1990
(51) Int. Cl.: G01N 15/08

(54) **Dispositif et méthode d'évaluation de l'aptitude qu'a un corps à s'opposer au passage d'un produit et leur application à l'évaluation de la dysmigration**
Verfahren und Vorrichtung zur Auswertung der Permeabilität eines Körpers und ihre Verwendung in der Auswertung der Dysmigration
Method and apparatus for evaluating the permeability of a body and their use in the evaluation of dysmigration

(30) Priorité: 28.12.1989 FR 8917519
(43) Date de publication de la demande: 17.07.1991
(73) Titulaire: INSTITUT FRANCAIS DU PETROLE, F-92502 Rueil-Malmaison (FR)
(72) Inventeur: Lafargue, Eric, F-75013 Paris (FR); Espitalie, Jean, F-78110 Le Vesinet (FR); Lesage, Thierry, F-78250 Tessancourt sur Aubette (FR)

(56) Documents cités:
- CA-A- 1 201 906
- US-A- 4 304 122
- US-A- 4 672 840
- US-A- 4 679 421
- REVIEW OF SCIENTIFIC INSTRUMENTS, vol. 53, no. 8, août 1982, pages 1246-1254, American Institute of Physics, New York, US; D. TRIMMER: "Laboratory measurements of ultralow permeability of geologic materials"
- PATENT ABSTRACTS OF JAPAN, vol. 8, no. 275 (P-321)[1712], 15 décembre 1984; & JP-A-59 142 438

## Description

La présente invention concerne l'évaluation de l'aptitude qu'a un corps à s'opposer au passage d'un produit. Elle s'applique particulièrement bien aux roches-couvertures de gisements pétroliers naturels.

L'aptitude d'une roche-couverture à s'opposer au passage d'hydrocarbures est un paramètre essentiel dans l'évaluation pétrolifère de formations géologiques. Un bonne roche-couverture s'opposera au passage des hydrocarbures à l'inverse une mauvaise roche-couverture sera facilement traversée par les hydrocarbures. Il n'existe pas à l'heure actuelle de moyen permettant de définir cette propriété de manière simple et fiable. La présente invention propose une méthode et un dispositif permettant notamment de classer les roches-couvertures en fonction de leur aptitude à s'opposer au passage des hydrocarbures dans les conditions d'un test de dysmigration réalisé en laboratoire sur des échantillons de roche. On entend par dysmigration le phénomène physique de fuite d'un produit tel un hydrocarbure d'un réservoir à travers une roche-couverture.

On connait le document CA-A-1.201.906 qui décrit une méthode et un appareil pour mesurer les qualités d'un cake déposé par un fluide de forage. On connait également le document US-A-4.672.840 qui concerne une méthode et un système pour déterminer la perméabilité d'un échantillon de roche en mesurant les volumes des fluides traversant l'échantillon. Dans ces deux documents, il n'est pas question de mesurer l'aptitude qu'à un corps à s'opposer au passage d'un produit en plaçant le corps entre deux réservoirs en matériau poreux.

Ainsi d'une manière générale la présente invention concerne une méthode permettant l'évaluation de l'aptitude qu'a un corps à s'opposer au passage d'un produit. Cette méthode comporte les étapes suivantes :
- on place ledit corps entre deux réservoirs constitués d'un matériau poreux, l'un étant un réservoir émetteur contenant ledit produit et l'autre étant le réservoir récepteur de la partie du produit ayant traversé ledit corps, ledit corps séparant lesdits réservoirs et étant adjacent à chacun d'eux.
- on réalise un gradient de pression entre lesdits réservoirs, ce gradient provoquant le déplacement vers ledit réservoir récepteur d'au moins une partie dudit produit contenu dans le réservoir émetteur à travers ledit corps, et
- on analyse la partie du produit récupéré dans ledit réservoir.

On pourra effectuer l'analyse de la partie du produit récupéré dans ledit réservoir récepteur par un balayage en continu dudit réservoir.

La méthode selon l'invention pourra comporter une étape de mise en pression dudit corps accompagnée éventuellement d'une opération de chauffage dudit corps.

L'analyse mentionnée ci-dessus pourra être une analyse qualitative et/ou quantitative de la partie récupérée dudit produit.

La méthode selon l'invention pourra comporter la détermination d'un critère de dysmigration lié à la quantité de produit récupéré dans le réservoir récepteur rapporté au volume dudit corps.

La méthode et le dispositif selon l'invention pourront être appliqués à l'évaluation de la dysmigration d'un hydrocarbure à travers un échantillon de roche. Ils pourront de même être appliqués à à l'évaluation de la dysmigration de produit autre que des hydrocarbures notamment l'eau, le monoxyde de carbone, le dioxyde de carbone ou au dioxyde de soufre.

La présente invention concerne également un dispositif permettant l'évaluation de l'aptitude qu'a un corps à s'opposer au passage d'un produit, ledit dispositif comportant une enceinte haute pression comportant elle-même un milieu de confinement, et des moyens de génération de pression de confinement. Il comporte, en outre, un ensemble, ou éprouvette, hermétiquement isolée du milieu de confinement, ledit ensemble comportant un échantillon dudit corps et deux réservoirs constitués d'un matériau poreux. Il comporte aussi des moyens de création d'un gradient de pression entre les deux réservoirs, l'un émetteur contenant ledit produit et l'autre récepteur destiné à récupérer la partie de produit ayant traversé ledit échantillon sous l'effet du gradient, lesdits réservoirs étant placés de part et d'autre dudit échantillon et étant adjacents audit échantillon.

Le dispositif pourra comporter des moyens de contrôle du gradient de pression existant entre ledit réservoir émetteur et ledit réservoir récepteur.

Le dispositif pourra comporter des moyens de balayage dudit réservoir récepteur.

Les moyens de balayage pourront comporter des moyens de détection et/ou d'analyse des fluides recueillis par balayage dans ledit réservoir récepteur.

Le dispositif selon l'invention pourra comporter des moyens de régulation en température des fluides de balayage.

Le dispositif selon l'invention peut comporter principalement une enceinte, ou cellule haute pression, dans laquelle est placé un assemblage constitué par la superposition ou la juxtaposition d'un réservoir émetteur d'un échantillon de roche et d'un réservoir récepteur au sein d'un ensemble isolé hermétiquement du milieu de confinement, de préférence par une jaquette métallique et deux bouchons en acier. Dans la suite de ce texte, on pourra également désigner cet ensemble par le terme d'éprouvette. Sous l'effet d'un gradient de pression entre les réservoirs et en présence d'une pression de confinement, les fluides ou produits contenus dans le réservoir émetteur se déplacent vers le réservoir récepteur où éventuellement un système de balayage peut assurer leur transport vers des détecteurs spécifiques, permettant ainsi une analyse en continu des produits expulsés.

L'originalité de la présente invention réside, notamment, dans la juxtaposition de l'échantillon de roche-couverture et des réservoirs, de part et d'autre de cet échantillon, dans un ensemble hermétique relativement au milieu de confinement. De plus, pour certains modes particuliers de réalisation, elle réside en outre dans l'analyse en continu des produits récupérés grâce au dispositif de balayage. Dans ce dernier cas, il est donc possible d'étudier la cinétique de la dysmigration à travers la roche-couverture, ce qui ne serait pas possible si on avait à démonter la cellule après chaque expérience. Ainsi on peut étudier la dysmigration d'un mélange gaz liquide à travers une roche-couverture. Par exemple un mélange d'hydrocarbure gazeux et liquide.

Bien entendu la présente invention permet également de caractériser l'aptitude qu'a un corps à être traversé par un produit.

La présente invention sera mieux comprise et ses avantages apparaitront plus clairement à la description qui suit d'un exemple particulier de mise en oeuvre du dispositif selon l'invention, nullement limitatif, appliqué au cas de roches, illustré par les figures ci-annexées.
- la figure 1 illustre dans son ensemble le dispositif selon l'invention,
- la figure 2 représente schématiquement la manière dont les pressions s'appliquent sur l'échantillon de roche et sur les réservoirs.

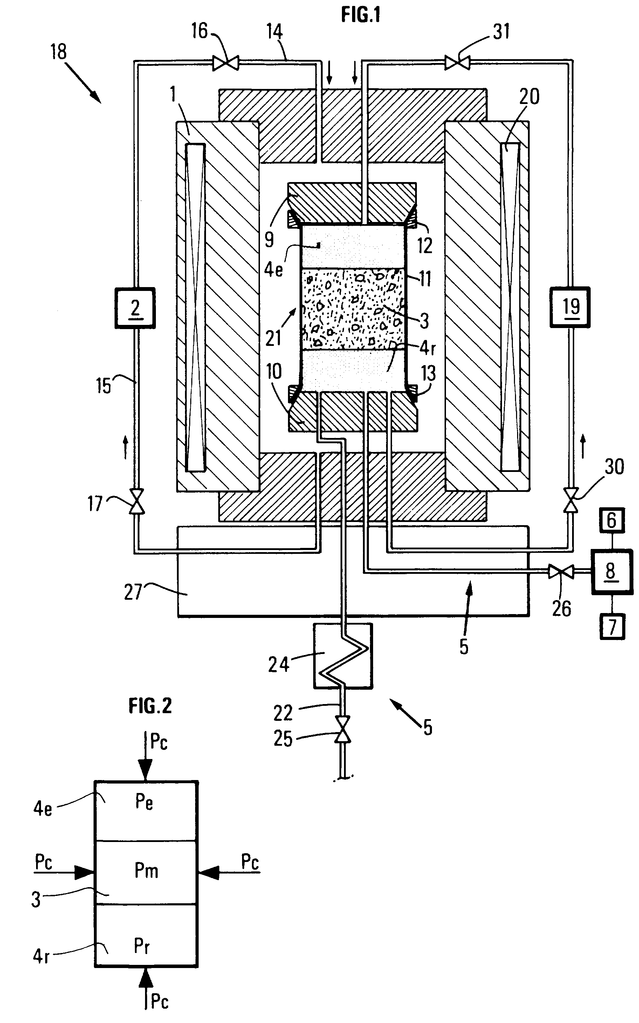

L'appareil illustré à la figure 1 comprend:
- un corps d'enceinte 1 haute pression, équipé d'un four 20, permettant de travailler à des pressions généralement comprises entre 1 et 4.000 bar et des températures généralement comprises entre 20 et 400°C, le four pouvant être intégré ou non au corps de l'enceinte,
- des moyens de génération de pression de confinement 2, et
- un ensemble 21 hermétiquement isolé du milieu de confinement lors des essais, cet ensemble ou éprouvette recevant l'échantillon de roche 3 à tester et un réservoir émetteur 4e et un réservoir récepteur 4r.

Le dispositif selon l'invention peut également comporter:
- des moyens de contrôle du gradient de pression 18, 19 entre le réservoir émetteur 4e et le réservoir récepteur 4r,
- des moyens de balayage des produits récupérés 5 dans le réservoir récepteur 4r, et/ou
- des détecteurs spécifiques aux hydrocarbures 6, et éventuellement aux CO₂, CO ET H₂O, 7, reliés à une boucle d'échantillonnage 8.

L'éprouvette 21 contient l'échantillon de roche 3 et les réservoirs émetteur 4e et récepteur 4r qui sont maintenus juxtaposés et isolés du milieu de confinement par l'intermédiaire de deux bouchons métalliques 9 et 10 et d'une jaquette métallique 11 sertie sur les précédents à l'aide de deux bagues métalliques 12 et 13, ayant éventuellement une section droite en forme de coin.

Les moyens de génération d'une pression de confinement 2 sont reliés à l'enceinte par des canalisations 14 et 15 qui comportent des vannes 16 et 17. Ces vannes permettent d'isoler le milieu de confinement des moyens 2 de génération de pression de confinement et éventuellement la vidange de l'enceinte de confinement lors de l'arrêt des moyens 2 de génération de la pression de confinement.

La référence 18 désigne dans leur ensemble les moyens servant à contrôler et éventuellement à créer le gradient de pression au sein de l'éprouvette 21. Ces moyens peuvent comporter une pompe et éventuellement des moyens de vannage 30, 31, notamment pour isoler l'espace interne de l'éprouvette de la pompe 19.

Les moyens 18 permettant de contrôler le gradient de pression entre les réservoirs 4e et 4r comportent de manière classique un capteur différentiel de pression relié à un système de régulation de la pression commandant une pompe pneumatique. Ces moyens 18 de contrôle du gradient sont désignés hors les canalisations et les moyens de vannage par la référence 19. Ces moyens 18 sont différents des moyens de génération de la pression de confinement.

Les moyens de génération de la pression de confinement peuvent comprendre une ou des pompes pneumatiques tout-à-fait classiques associées à des systèmes de régulation reliés à un capteur de pression mesurant la pression régnant dans l'enceinte de confinement.

Les moyens de balayage 5 peuvent comporter une source de fluide de balayage non représentée et un circuit d'entrée 22 amenant le fluide de balayage fourni par cette source au réservoir récepteur 4r, à travers le bouchon 10 et un circuit de sortie 23 menant le fluide de balayage vers la boucle d'échantillonnage 8, après que celui-ci ait traversé le réservoir récepteur 4r.

Le circuit d'entrée 22 peut comporter des moyens de chauffage permettant de réchauffer le fluide de balayage.

Le circuit de balayage comporte des vannes 25 et 26 permettant d'interrompre la circulation du fluide de balayage.

Pour éviter la condensation dans la canalisation du circuit de sortie 23 des moyens de balayage, celle-ci pourra cheminer au moins sur une grande partie de sa longueur dans un ensemble thermostaté 27. Les autres canalisations pourront également cheminer en partie dans lesdits moyens thermostatés, comme cela est représenté à la figure 1.

Le dispositif selon l'invention permet de réaliser des tests de dysmigration sur des échantillons de roches-couvertures naturelles, carottes ou plugs. Le diamètre de ces échantillons pourra être compris entre 20 et 50 mm pour une longueur comprise aussi entre 20 et 55 mm.

Le test de dysmigration consiste à étudier la quantité éventuellement la nature et la composition de produits récupérés dans le réservoir récepteur au cours du temps dans les conditions de pression et de température du test. Les réservoirs 4e et 4r pourront être en roche naturelle (grès, carbonates...), ou en matériau poreux quelconque (fritte métallique, céramique...). Leurs épaisseurs pourront être égales ou pas et varier par exemple entre 2 et 20 mm. Le réservoir récepteur 4r peut être vide à l'origine, ou contenir un fluide tel de l'air ou de l'eau, ce fluide étant de préférence différent de celui contenu dans le réservoir émetteur. La présence d'un fluide dans le réservoir récepteur permet de contrôler la pression qui y règne. Dans le mode de réalisation décrit à la figure 1, les produits récupérés dans le réservoir récepteur 4r, sont acheminés vers les différents détecteurs 6 et 7 par l'intermédiaire d'un système de balayage faisant intervenir un fluide chauffé (gaz inertes, eau avec ou sans tensio-actifs, CO₂, solvants organiques). L'ensemble est chauffé grâce à un système thermostaté 27, afin d'éviter l'apparition de points froids sur le circuit de balayage de sortie 23 où pourraient se condenser les produits, faussant ainsi les analyses quantitatives. A la sortie de la boucle d'échantillonnage 8, les hydrocarbures, l'eau, le CO, le CO₂ et l′H₂S sont analysés séparément.

Les gammes de température et de pression peuvent être étendues de 20 à 400°C et de 1 à 4.000 bar. Les pressions et les températures peuvent être enregistrées automatiquement et peuvent être programmées en fonction du temps.

Comme cela a été dit, le dispositif expérimental permet de déterminer l'aptitude d'une roche à s'opposer du passage de fluides notamment les hydrocarbures, l'eau, le CO, le CO₂ et l′H₂S. Il est possible, selon la présente invention, de classer les roches-couvertures en roches-couvertures très bonnes, ne laissant pas passer facilement les hydrocarbures, par opposition aux mauvaises roches-couvertures, laissant passer de façon notable les hydrocarbures.

On peut également selon la présente invention déterminer la cinétique de la dysmigration à partir de l'analyse en continu des produits récupérés dans le réservoir 4r ce qui permet aussi de caractériser l'aptitude des roches testées à laisser passer les produits accumulés dans les réservoirs naturels en fonction du temps.

La figure 2 illustre schématiquement les pressions auxquelles sont soumis l'échantillon 3 et les réservoirs 4r et 4e.

Sur cette figure, Pc représente la pression de confinement qui s'applique au squelette de l'échantillon de roche 3 et à celui des réservoirs 4r et 4e. Cette pression Pc correspond sensiblement à celle qui règne dans l'enceinte, du moins lorsque l'effort de résistance de la jaquette métallique est négligeable par rapport aux efforts créés par la pression de confinement.

Pm désigne la pression au sein des pores de la roche-couverture et Pe et Pr désignent respectivement les pression régnant au sein des pores du réservoir émetteur et du réservoir récepteur.

Le gradient de pression auquel sont soumis les réservoirs correspond à la différence: Pe - Pr. C'est ce gradient qui permet de faire passer les fluides à travers l'échantillon de roche 3.

Dans les conditions normales d'essai on impose Pe > Pm > Pr.

On étudie la quantité de fluide initialement contenu dans le réservoir émetteur et qui a traversé l'échantillon 3 pour se retrouver dans le réservoir récepteur 4r.

Le dispositif selon la présente invention permet de déterminer la qualité de roches-couvertures.

A titre d'exemple on peut définir un indice de dysmigration IDH proportionnel ou égal à la quantité de fluide initialement contenu dans le réservoir émetteur et ayant traversé l'échantillon de roche et qui est récupérée dans le réservoir récepteur.

Pour pouvoir comparer facilement plusieurs échantillons entre eux, il est préférable que l'IDH de chacun de ces échantillons soit déterminé dans des conditions de tests identiques prédéterminées. Ainsi on pourra fixer les différentes pressions: pression de confinement, pression dans les réservoirs et/ou gradient de pression. De même la température, la durée, les dimensions des différentes parties constitutives du dispositif et la nature des réservoirs seront fixées à l'avance.

La pression de confinement Pc prédéterminée pourra être comprise par exemple entre 1000 et 3000 bar, et le gradient de pression prédéterminé pourra être compris par exemple entre 500 et 2000 bar.

La température prédéterminée de l'enceinte pourra être par exemple comprise entre 50 et 200°C et de préférence entre 50 et 150°C.

La durée de maintien du gradient de pression pourra être par exemple comprise entre 24 heures et 168 heures et de préférence voisine ou égale à 72 heures.

Les réservoirs et l'échantillon de la roche pourront être cylindriques et avoir un diamètre prédéterminé par exemple compris entre 20 et 50 mm et avoir une hauteur comprise entre 20 et 55 mm.

Le rapport du volume prédéterminé de l'échantillon de roche à celui du réservoir émetteur pourra être compris éventuellement entre 0,5 et 1,5 et de préférence sensiblement égal à 1.

Le réservoir récepteur pourra avoir la même forme que le réservoir émetteur.

Le réservoir peut être une roche naturelle (grès, carbonate poreux, roche volcanique...), un matériel poreux de synthèse (fritte métallique, céramique...).

La jaquette pourra être réalisée en cuivre.

## Revendications

1. Méthode permettant l'évaluation de l'aptitude qu'a un corps (3) à s'opposer au passage d'un produit, caractérisée en ce qu'elle comporte les étapes suivantes :
- on place ledit corps (3) entre deux réservoirs (4e, 4r) constitués d'un matériau poreux, l'un étant un réservoir émetteur (4e) contenant ledit produit et l'autre étant le réservoir récepteur (4r) de la partie du produit ayant traversé ledit corps (3), ledit corps séparant lesdits réservoirs et étant adjacent à chacun d'eux.
- on réalise un gradient de pression entre lesdits réservoirs, ce gradient provoquant le déplacement vers ledit réservoir récepteur d'au moins une partie dudit produit contenu dans le réservoir émetteur à travers ledit corps, et
- on analyse la partie du produit récupéré dans ledit réservoir.

2. Méthode selon la revendication 1, caractérisée en ce que l'on effectue l'analyse de la partie du produit récupéré dans ledit réservoir récepteur (4r) par un balayage en continu dudit réservoir.

3. Méthode selon l'une des revendications 1 à 2, caractérisée en ce qu'elle comporte une étape de mise en pression dudit corps (3) accompagnée éventuellement d'une opération de chauffage dudit corps.

4. Méthode selon l'une des revendications précédentes, caractérisée en ce que ladite analyse comporte une opération d'analyse qualitative et/ou quantitative de la partie récupérée dudit produit.

5. Méthode selon la revendication 4, caractérisée en ce qu'elle comporte la détermination d'un critère de dysmigration lié à la quantité de produit récupéré dans le réservoir récepteur rapporté au volume dudit corps.

6. Application des méthodes des revendications 1 à 5 à l'évaluation de la dysmigration d'un hydrocarbure à travers un échantillon de roche.

7. Application selon la revendication 6 à l'évaluation de la dysmigration de produit autre que des hydrocarbures notamment l'eau, le monoxyde de carbone, le dioxyde de carbone ou au dioxyde de soufre.

8. Dispositif permettant l'évaluation de l'aptitude qu'a un corps (3) à s'opposer au passage d'un produit, ledit dispositif comportant une enceinte haute pression (1) comportant elle-même un milieu de confinement, et des moyens de génération de pression de confinement (2), caractérisé en ce qu'il comporte un ensemble (21) ou éprouvette hermétiquement isolée du milieu de confinement, ledit ensemble comportant un échantillon dudit corps (3) et deux réservoirs (4e, 4r) constitués d'un matériau poreux, des moyens de création (19) d'un gradient de pression entre lesdits deux réservoirs (4e, 4r), l'un émetteur contenant ledit produit et l'autre récepteur destiné à récupérer la partie de produit ayant traversé ledit échantillon sous l'effet dudit gradient, lesdits réservoirs étant placés de part et d'autre dudit échantillon et étant adjacents audit échantillon.

9. Dispositif selon la revendication 8, caractérisé en ce qu'il comporte des moyens de contrôle du gradient de pression (18) existant entre ledit réservoir émetteur et ledit réservoir récepteur.

10. Dispositif selon l'une des revendications 8 ou 9, caractérisé en ce que ledit dispositif comporte des moyens de balayage (5) dudit réservoir récepteur.

11. Dispositif selon la revendication 10, caractérisé en ce que lesdits moyens de balayage comportent des moyens de détection et/ou d'analyse (8, 6, 7) des fluides recueillis par balayage dans ledit réservoir récepteur.

12. Dispositif selon l'une des revendications 10 ou 11, caractérisé en ce qu'il comporte des moyens de régulation en température (24, 27) des fluides de balayage.

## Patentansprüche

1. Verfahren, das es ermöglicht, die Fähigkeit eines Körpers (3), sich dem Durchgang eines Produktes zu widersetzen, zu bewerten, dadurch gekennzeichnet, daß es die folgenden Stufen umfaßt:
- man ordnet diesen Körper (3) zwischen zwei Speichern (4e, 4r), die aus einem porösen Material gebildet sind, an, wobei einer ein abgebender dieses Produkt enthaltender Speicher (4e) und der andere der aufnehmende Speicher (4r) des Teiles des Produktes, das diesen Körper (3) durchsetzt hat, ist, wobei dieser Körper diese Speicher trennt und einem jeden von ihnen benachbart ist,
- man realisiert einen Druckgradienten zwischen diesen Speichern, wobei dieser Druckgradient die Verdrängung wenigstens eines Teils dieses im abgebenden Speicher enthaltenen Produktes durch diesen Körper hindurch zu diesem Aufnehmerspeicher hervorruft und
- man analysiert den Teil des in diesem Speicher gewonnenen Produktes.

2. Verfahren nach Anspruch 1, dadurch gekennzeichnet, daß man die Analyse des Teils des in diesem Aufnehmerspeicher (4r) gewonnenen Produktes durch eine kontinuierliche Spülung dieses Speichers vornimmt.

3. Verfahren nach einem der Ansprüche 1 bis 2, dadurch gekennzeichnet, daß es eine Stufe zur Unter-Druck-Setzung dieses Körpers (3), gegebenenfalls begleitet von einem Heizvorgang dieses Körpers umfaßt.

4. Verfahren nach einem der vorhergehenden Ansprüche, dadurch gekennzeichnet, daß diese Analyse eine qualitative und/oder quantitative Analysemaßnahme des rückgewonnenen Teils dieses Produktes umfaßt.

5. Verfahren nach Anspruch 4, dadurch gekennzeichnet, das es die Bestimmung eines Dysmigrationskriteriums, verknüpft mit der Menge an in diesem Aufnehmerspeicher gewonnenen Produktes, bezogen auf das Volumen dieses Körpers, umfaßt.

6. Anwendung der Verfahren der Ansprüche 1 bis 5 auf die Bewertung der Dysmigration eines Kohlenwasserstoffes durch eine Gesteinsprobe.

7. Anwendung nach Anspruch 6, auf die Bewertung der Produktdysmigration außer Kohlenwasserstoffen, insbesondere des Wassers, des Kohlenmonoxyds, des Kohlendioxyds oder des Schwefeldioxyds.

8. Vorrichtung, die es ermöglicht, die Fähigkeit eines Körpers (3), sich dem Durchgang eines Produktes zu widersetzen, zu bewerten, wobei die Vorrichtung eine Hochdruckkammer (1) umfaßt, die selbst ein Begrenzungsmedium und Mittel zur Erzeugung des Begrenzungsdrucks (2) umfaßt, dadurch gekennzeichnet, daß sie eine Anordnung (21) oder einen hermetisch vom Begrenzungsmedium isolierten Prüfkörper umfaßt, wobei die Anordnung eine Probe dieses Körpers (3) und zwei Speicher (4e, 4r) umfaßt, die aus einem porösen Material gebildet sind, Mittel (19) zur Erzeugung eines Druckgradienten zwischen diesen beiden Speichern (4e, 4r), wobei der eine, der abgebende Speicher dieses Produkt enthält und der andere der aufnehmende Speicher dazu bestimmt ist, den Teil des Produktes rückzugewinnen, der diese Probe unter dem Einfluß dieses Gradienten durchsetzt hat, wobei diese Speicher zu beiden Seiten dieser Probe angeordnet und benachbart dieser Probe vorgesehen sind.

9. Vorrichtung nach Anspruch 8, dadurch gekennzeichnet, daß sie Mittel zum Regeln des Druckgradienten (18), der zwischen diesem abgebenden Speicher und diesem aufnehmenden Speicher existiert umfaßt.

10. Vorrichtung nach einem der Ansprüche 8 oder 9, dadurch gekennzeichnet, daß diese Vorrichtung Mittel (5) zum Spülen dieses aufnehmenden Speichers umfaßt.

11. Vorrichtung nach Anspruch 10, dadurch gekennzeichnet, daß diese Spülmittel Mittel der Erfassung und/oder Analyse (8, 6, 7) der Fluide umfassen, welche sich durch Spülen in diesem aufnehmenden Speicher gesammelt haben.

12. Vorrichtung nach einem der Ansprüche 10 oder 11, dadurch gekennzeichnet, daß sie Mittel zur Temperatursteuerung (24, 27) der Spülfluide umfaßt.

## Claims

1. Method enabling the aptitude of a body (3) to resist the passage of a product to be assessed, characterised in that it comprises the following steps:
- the said body (3) is placed between two reservoirs (4e, 4r) of a porous material, one being a transmitting reservoir (4e) containing the product and the other being the reservoir that will receive (4r) the proportion of the product that has passed through the said body (3), the said body separating the said reservoirs and being adjacent to each of them.
- a pressure gradient is produced between the said reservoirs, this gradient causing the displacement of at least a proportion of the said product contained in the transmitting reservoir through the body and to the said receiving reservoir, and
- the portion of the product recovered in this reservoir is analyzed.

2. Method as claimed in claim 1, characterised in that the portion of product recovered in the said receiving reservoir (4r) is analyzed by continuously purging this said reservoir.

3. Method as claimed in one of claims 1 to 2, characterised in that it comprises a step in which the body (s) is put under pressure, possibly accompanied by a process during which the said body is heated.

4. Method as claimed in one of the previous claims, characterised in that the said assessment includes a qualitative and/or quantitative analysis of the recovered portion of the said product.

5. Method as claimed in claim 4, characterised in that it involves determining a criterion of dysmigration linked to the quantity of product recovered in the receiving reservoir relative to the volume of the said body.

6. Application of the methods of claims 1 to 5 to the assessment of dysmigration of a hydrocarbon through a rock sample.

7. Application as claimed in claim 6 to the assessment of dysmigration of a product other than hydrocarbons, in particular water, carbon monoxide, carbon dioxide or sulphur dioxide.

8. Device enabling the aptitude of a body to resist the passage of a product to be assessed, the said device comprising a high pressure chamber (1), itself constituting a confinement medium, and means for generating a confinement pressure (2), characterised in that it has an assembly (21) or test cell hermetically isolated from the confinement medium, the said assembly comprising a sample of the said body (3) and two reservoirs (4r, 4e) of a porous material, means for creating (19) a pressure gradient between the two reservoirs (4e, 4r), the transmitting reservoir containing the said product and the other receiving reservoir being used to recover the portion of the product that has passed through the said sample under the effect of the said gradient, the said reservoirs being placed on either side of the said sample and being adjacent to the said sample.

9. Device as claimed in claim 8, characterised in that it has means for controlling the pressure gradient (18) existing between the said transmitting reservoir and the said receiving reservoir.

10. Device as claimed in one of claims 8 or 9, characterised in that the said device has means for purging (5) the said receiving reservoir.

11. Device as claimed in claim 10, characterised in that the said purging means have means for detecting and/or analyzing (8, 6, 7) the fluids picked up by the purging process in the receiving reservoir.

12. Device as claimed in one of claims 10 or 11, characterised in that it comprises means for regulating the temperature (24, 27) of the purging fluids.
